Europäisches Patentamt

European Patent Office

Office européen des brevets

(19)

(11) Veröffentlichungsnummer: **0 185 939 B1**

(12) **EUROPÄISCHE PATENTSCHRIFT**

(45) Veröffentlichungstag der Patentschrift: **16.08.90**

(21) Anmeldenummer: **85114841.1**

(22) Anmeldetag: **22.11.85**

(51) Int. Cl.⁵: **A 61 M 36/12,** A 61 N 5/10

(54) **Ferngesteuerte Afterloading Vorrichtung insbesondere zur Brachycurie-Therapie von Tumoren.**

(30) Priorität: **23.11.84 DE 3442762**

(43) Veröffentlichungstag der Anmeldung:
**02.07.86 Patentblatt 86/27**

(45) Bekanntmachung des Hinweises auf die
Patenterteilung:
**16.08.90 Patentblatt 90/33**

(84) Benannte Vertragsstaaten:
**AT CH FR GB LI NL SE**

(56) Entgegenhaltungen:
**DE-A-1 945 015**
**DE-A-2 717 341**
**DE-A-2 727 359**
**DE-A-3 313 857**
**DE-B-1 764 688**
**US-A-3 861 380**
**US-A-3 866 050**

(73) Patentinhaber: **Puthawala, Anwer, Dipl.-Ing.**
**Weiselstrasse 46**
**D-8520 Erlangen-Buckenhof (DE)**

(72) Erfinder: **Puthawala, Anwer, Dipl.-Ing.**
**Weiselstrasse 46**
**D-8520 Erlangen-Buckenhof (DE)**
Erfinder: **Puthawala, Ajmel, Dr.**
**39 Country Lane**
**Rolling Hills Estate CA 90274 (US)**

Courier Press, Leamington Spa, England.

EP 0 185 939 B1

**Beschreibung**

Die Erfindung bezieht sich auf eine Vorrichtung zur Strahlenbehandlung von Tumoren. Diese ferngesteuerte Vorrichtung schließt jeden ungewollten Kontakt mit radioaktivem Material aus und bietet vollständigen Strahlenschutz für Arzt und Personal. Durch das automatische Be- und Entladen der Strahlenquellen wird die manuelle Arbeit mit der Strahlenquelle eliminiert. Nach Beladen der Strahlenquelle kann sich der Patient selbst oder vom Arzt von der Afterloading Vorrichtung entkoppeln.

Unter Brachycurie-Therapie werden interstielle und intrakavitäre Therapie verstanden. Bei der Brachycurie-Therapie werden zunächst mehrere Plastikkatheter oder Hohlnadeln in und um den Tumor implantiert und anschließend umschlossene Strahler, z.B. Iridium oder Cesium in den Plastikkatheter oder in die Hohlnadeln eingeführt und von außen festgehalten.

Bei der Brachycurie-Therapie werden zwei Methoden, nämlich Kurzzeitbestrahlung (high dose rate) und Langzeitbestrahlung (low dose rate) angewandt. Die Kurzzeitbestrahlung wird mit entsprechend starker Strahlungsquelle in relativ kurzer Zeit, z.B. ca. 30 Min. durchgeführt. Dagegen wird bei der Langzeitbestrahlung mit entsprechend geringerer Strahlungsquelle in längerem Zeitraum, z.B. 1 bis 5 Tage die Behandlung durchgeführt.

Die für die Kurzzeitbestrahlung (high dose rate) angewandten Afterloading Vorrichtungen sind z.B. aus US-Patent Nr. 3 861 380 und Offenlegungsschrift DE-A-1945 015 bekannt.

Die bekannten Vorrichtungen sind jedoch für eine Langzeitbestrahlung aus mehreren Gründen in der Praxis nicht angewandt. Der Hauptnachteil der bekannten Vorrichtungen ist, daß nach Beladen der Strahlungsquelle im Patienten, diese Vorrichtungen vom Patienten nicht entkoppelbar sind. Dies bedeutet, daß der Patient während der gesamten Bestrahlungsdauer, in vielen Fällen bis zu mehreren Tagen, ständig mit der Vorrichtung verbunden sein muß. Die Langzeitbestrahlung erfordert in vielen Fällen gleichzeitig mehrere Strahlungsquellen, beispielsweise bis zu 48 Kanäle. Die bekannten Vorrichtungen für die Kurzzeitbestrahlung haben jedoch weniger Kanäle. Aus diesen Gründen wird zur Zeit weltweit bei der Langzeitbestrahlung das Be- und Entladen der Strahler unter Teilabschirmung manuell durchgeführt. Die Strahlungsquelle wird von Hand aus einem Abschirmbehälter entnommen, und in den im Patienten implantierten Plastikkatheter oder Hohlnadel eingeführt und am Ende der Bestrahlungszeit wieder entnommen. Der behandelnde Arzt ist dabei nur durch unzureichende Bleiabschirmung geschützt. Dies führt zur erhöhten Strahlenbelastung, insbesondere der Augen und Hände der Operateure und des Personals.

Aufgabe der Erfindung ist das automatische, ferngesteuerte Be- und Entladen der Strahler, sowie das manuelle Entkoppeln der Strahler von der Vorrichtung nach Beladen, und wieder Ankoppeln vor dem Entladen, wobei das manuelle Ent- und Ankoppeln selbst von dem Patienten ausgeführt werden kann. Gelöst wird die gestellte Aufgabe durch die im Kennzeichnungsteil des Anspruches 1 angegebenen Merkmale. Vorteilhafte Weiterbildungen der Erfindung sind in den Unteransprüchen 2 bis 10 angegeben.

Die mit der Erfindung erzielten Vorteile bestehen insbesondere darin, daß der Patient während der gesamten Bestrahlungsdauer frei beweglich ist, weil er nicht mit der Vorrichtung verbunden ist. Durch das ferngesteuerte, mit Magneten einfach und sicher durchgeführte Be- und Entladen der Strahler werden Arzt und Personal vor Strahlung geschützt.

Nachstehend wird ein Ausführungsbeispiel der Vorrichtung nach der Erfindung anhand der Zeichnungen beschrieben. Es zeigt.

Fig. 1: Eine Schnittansicht einer ferngesteuerten, fahrbaren Afterloading Vorrichtung nach der Erfindung.

Fig. 2: Ribbon in Rohr 9, mit Strahler (seeds genannt), Plastikröhrchen und magnetischem Betätigungelement (Nadel) nach der Erfindung.

Fig. 3: Vordere Ansicht der in Fig. 1 dargestellten Vorrichtung mit geöffnetem Deckel nach der Erfindung.

Fig. 4: Eine Schnittansicht eines Kupplungs-Schiebers, welcher an der obengenannten Vorrichtung (Fig. 1) an- und entkoppelt werden kann nach der Erfindung.

Fig. 5: Vorere Ansicht des in Fig. 4 dargestellten Kupplungsschiebers nach der Erfindung.

Fig. 6A/B/C: Schematische Darstellung der formschlüssigen Arretierung von Ribbon.

Fig. 7: Schematische Darstellung der ferngesteuerten Afterloading Vorrichtung integriert in einem Wagen.

Die Afterloading Vorrichtung kann auch als Transportbehälter zum Transportieren der Strahler verwendet werden.

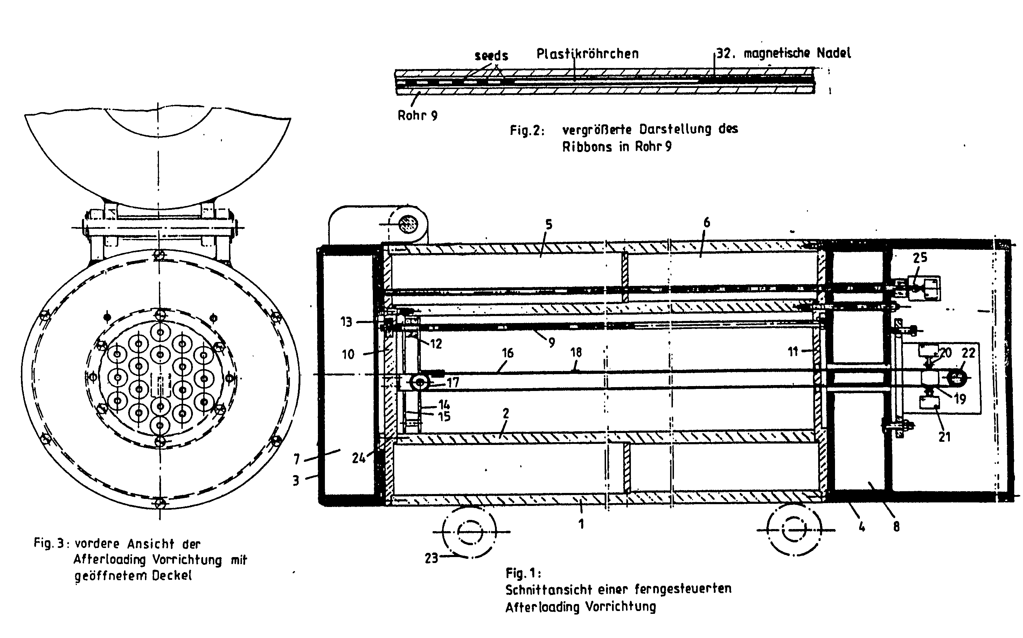

Wie Fig. 1 zeigt, besteht die Vorrichtung aus zwei ineinander coaxial angeordneten Röhren 1 und 2, welche an ihren vorderen Seiten mit einem beweglichen Deckel 3 und an ihren hinteren Seiten mit einem normalerweise fest verschlossenen Deckel 4 verbunden sind. Vorzugsweise bestehen alle Teile aus nicht magnetisierbarem Material. Die Hohlräume 5, 6, 7 und 8 sind ebenfalls mit nicht magnetisierbarem Abschirmmaterial gefüllt, vorzugsweise Bleikugeln mit unterschiedlichem Durchmesser oder Bleiwolle.

Im inneren Rohr 2 sind je nach Bedarf, ein oder mehrere kleine Rohre 9 aus nicht magnetisierbarem Material bestehend, parallel zueinander angeordnet, von denen in der Schnittzeichnung jedoch nur eines dargestellt ist. Dieses Rohr 9 ist an seiner vorderen Seite offen und ist durch die Platte 10 durchgesteckt. Die hintere Seite des Rohres 9 ist durch die Platte 11 durchgesteckt, jedoch außen verschlossen. Der innere Durchmesser des Rohres 9 muß größer sein als der Durchmesser des Strahlers.

Ein Strahler, nachfolgend Ribbon genannt, enthält wie Fig. 2 in vergrößertem Maßstab zeigt, auf einer Seite mehrere radioaktive Seeds, welche in einem biegsamen Plastikröhrchen untergebracht sind, um die Kontamination zu verhindern.

Wie die oben genannte Figur weiterhin zeigt, ist auf der anderen Seite von Seeds eine magnetisierbare Nadel, zum Beispiel aus Eisen, mit dem Plastikröhrchen fest verbunden. Die magnetische Nadel ist notwendig, um eine lineare Bewegung des Ribbons mit Hilfe eines Magneten durchzuführen. Fig. 1 zeigt, ein bzw. mehrere Ringmagneten 12, vorzugsweise aus Samarium-Cobalt Material bestehend, gleiten auf dem Rohr 9 ohne Schmiermittel. Statt dieser Permanent-Magneten können auch elektrische Magneten auf Rohr 9 angebracht werden, um eine lineare Bewegung des Ribbons mit magnetischer Nadel zu erzielen. Anstelle dieser einzelnen Ringmagneten 12, können auch andere geometrische Formen des Magneten eingesetzt werden.

Weiterhin zeigt Fig. 1, daß in der Platte 10, auf dem durchgesteckten Rohr 9 auch ein oder mehrere Ringmagnete 13 angebracht sind. Wie Fig. 3 auf der linken Seite zeigt, ist die vordere Ansicht der Vorrichtung mit geöffnetem Deckel dargestellt und als Beispiel sind 18 Rohre 9 mit Ringmagnete 13 eingezeichnet.

Um die Magneten 12 gleichzeitig bewegen zu können, sind sie zwischen zwei Platten 14 und 15, vorzugsweise aus nicht magnetisierbarem Material, eingespannt. Die Platte 14 ist mit einem Zahnriemen 16 verbunden. Ein Zahnrad 17 ist wiederum mit der platte 10 verbunden und ragt durch die Öffnungen der Platten 14 und 15. Der Zahnriemen 16 ist im Zahnrad 17 eingelegt.

Zwei Nocken 18 und 19 sind auf dem Zahnriemen 16 befestigt, um mit den Endschaltern 20 und 21 den Antrieb abzuschalten. Das Zahnrad 22 ist mit dem Antrieb verbunden, wobei der Antrieb selbst nicht in der Fig. 1 dargestellt ist. Mit der Bewegung des Zahnriemens werden die Platten 14 und 15 mit den eingespannten Magneten entlang des Rohrs 9 bewegt.

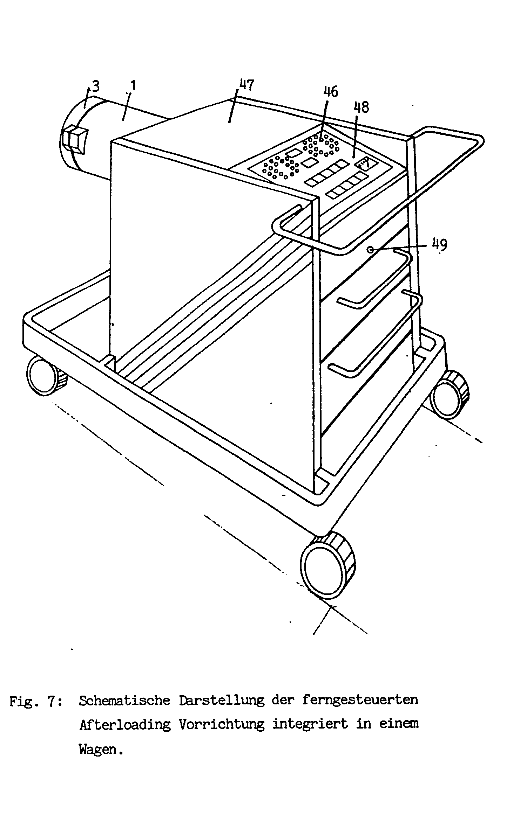

Die Fig. 1 zeigt, daß die Platten 14 und 15 an ihrer vorderen Endposition angekommen sind und durch Nocken 19 und Endschalter 21 der Antrieb automatisch abgeschaltet wurde. Die Geschwindigkeit des Antriebs kann je nach benötigter Zeit zum Be- und Entladen des Ribbons eingestellt werden. Die lineare Bewegung der Platten 14 und 15 kann auch beispielsweise durch Kugelumlaufspindel erzielt werden. Im Falle eines Stromausfalls kann der Antrieb auch manuell bedient werden. Die Betätigung des Antriebes erfolgt über ein separates Schaltpult (Figur 7), welches mit der Afterloading Vorrichtung verbunden ist. Alle für die Strahlentherapie notwendigen Daten, wie Beladedauer, Gesamtdosis, Anzahl und Zeitpunkt der Ankopplungs- und Entkopplungsvorgänge, können auf dem Schaltpult registriert werden. Um die gesamte Vorrichtung transportieren zu können, sind an der Unterseite mehrere Räder 23 angebracht.

Die Bohrungen 24 dienen zum Ankoppeln des Kupplungs-Schiebers an die Afterloading Vorrichtung, dabei wird über einen Kontaktstift (nicht dargestellt) der Endschalter 25 zum Ansprechen gebracht.

Dies bedeutet, daß der Kupplungs-Schieber ordnungsgemäß an die Afterloading Vorrichtung angeschlossen ist.

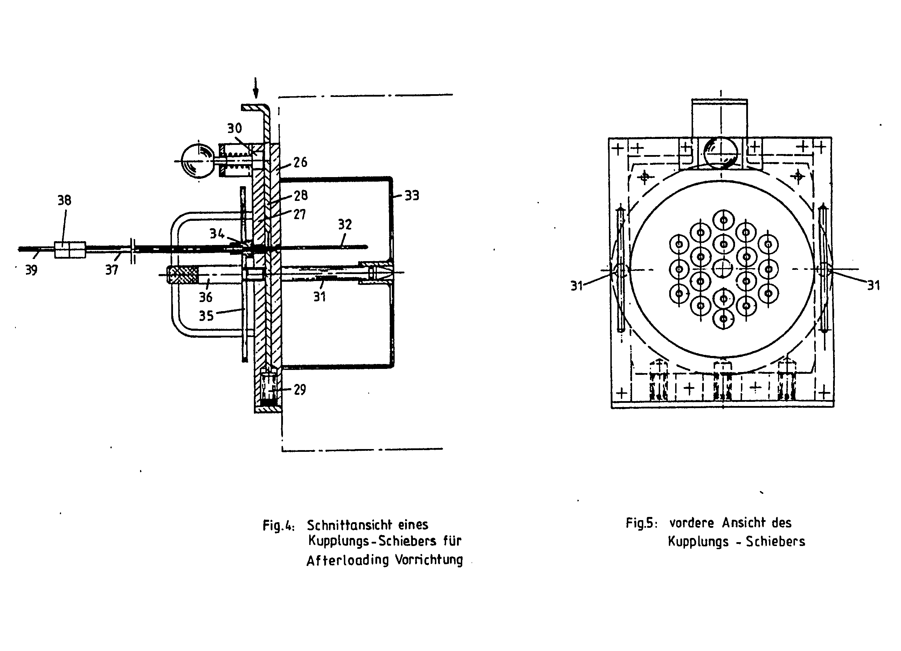

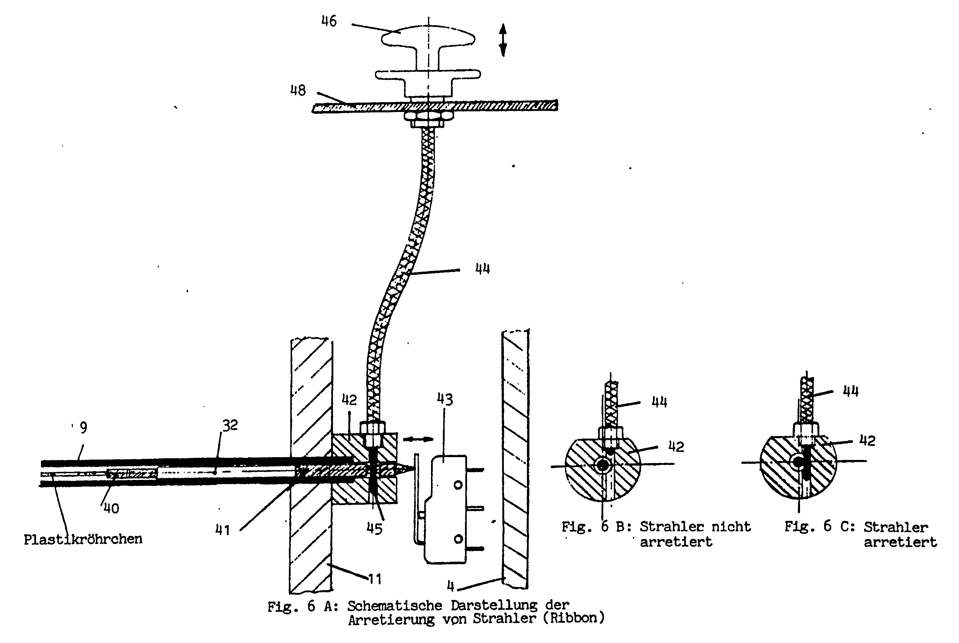

Der in Fig. 4 und 5 gezeigte Kupplungs-Schieber verbindet über geeignete biegsame Plastikschläuche den Patienten mit der Afterloading Vorrichtung. Die Kanäle (Rohr 9), die für die Strahlentherapie nicht benötigt werden, können mit dem Kupplungs-Schieber blind gemacht werden. Nach Beladen der Ribbon im Patienten werden die magnetischen Nadeln durch die mittlere Platte des Kupplungs-Schiebers festgehalten, um den Kupplungs-Schieber von der Afterloading Vorrichtung zu entkoppeln.

Das Gewicht des Kupplungs-Schiebers ist relativ niedrig, beispielsweise 700 gr, so daß er mit Hilfe eines Gürtels vom Patienten selbst getragen werden kann.

Der Kupplungs-Schieber besteht aus drei dünnen Platten mit Bohrungen, vorzugsweise aus Aluminium. Die Bohrungen der zwei äußeren Platten 26 und 27 stimmen mit denen in Fig. 1 beschriebenen Rohr (9)-Innendurchmesser überein. Diese beiden Platten 26 und 27 sind miteinander verschraubt. Die mittlere Platte 28 ist beweglich. Sie liegt unten auf mehreren Federn 29 auf und ragt oben etwas heraus. Sie hat größere Bohrungen als die Platten 26 und 27. In die Bohrungen sind dünne Gummiringe (nicht dargestellt) eingelegt. Wenn diese Platte 28 heruntergedrückt wird, rastet der Stift 30 automatisch in diese Platte ein. Damit ist der Kupplungs-Schieber offen.

Der Kupplungs-Schieber enthält zwei Zentrierstifte 31 mit unterschiedlichem Durchmesser, um nur eine Verbindungsmöglichkeit mit der Afterloading Vorrichtung zu gewährleisten. Um diese Verbindung herzustellen, muß zunächst der Deckel 3 geöffnet werden und die Zentrierstifte 31 in die dafür vorgesehenen Bohrungen 24 einzustecken.

Die Fig. 4 zeigt, daß der Kupplungs-Schieber bereits von der Afterloading Vorrichtung abgekoppelt ist, die Schieberplatte 28 ist geschlossen und damit sind die magnetischen Nadeln 32 fixiert. Ein abnehmbarer Deckel 33, zum Schutze der Patienten vor den magnetischen Nadeln 32, ist mit Hilfe der Zentrierstifte 31 angebracht.

Die Verbindungsstifte 34 werden durch die Platte 35 und einem Gewindestift 36 an den Kupplungs-Schieber angeschlossen. Der flexible Schlauch 37 ist über ein Verbindungsstück 38 mit dem im Patienten implantierten Plastikkatheter 39 oder Hohlnadeln verbunden.

Durch den Kupplungs-Schieber können die nicht benötigten Kanäle (Anzahl des Rohres 9) blind gemacht werden.

Als Beispiel:

Die Afterloading Vorrichtung enthält 18 Kanäle (Anzahl des Rohres 9). Für eine angenommene Strahlentherapie werden die Kanäle 2, 4, 6,...... 18 nicht benötigt. Diese Kanäle werden mit besonderen Verschlußstiften (nicht dargestellt), die keine Bohrung enthalten, blind gemacht. Die anderen Kanäle 1, 3, 5,...... 17 sind offen, da die Verbindungsstifte 34 Bohrungen enthalten. Wenn in diesem Fall, der Kupplungs-Schieber an die Afterloading Vorrichtung angeschlossen ist, und der Antrieb eingeschaltet ist, werden alle Kanäle zunächst aktiviert, da sich die Magnete entlang der Röhre 9 von hinten nach vorne bewegen. Die Ribbons in den Kanälen 2, 4, 6,...... 18 können nicht in den Kupplungs-Schieber hineinkommen, weil diese Kanäle durch die Verschlußstifte verschlossen sind. Die anderen Ribbons 1, 3, 5, ......17 werden ungehindert durch den Kupplungs-Schieber zum Patienten transportiert. Beim Erreichen der Endpostion der Platten 14 und 15 wird der Antrieb automatisch abgeschaltet. Die magnetischen Nadeln der Kanäle 1, 3, 5, ......17, welche jetzt im Kupplungs-Schieber angekommen sind, werden durch Herausziehen des Stifts 30 durch die mittlere Platte 29 fixiert. Jetzt kann der Kupplungs-Schieber von der Afterloading Vorrichtung entkoppelt werden.

Ein weiteres Ausführungsbeispiel in der Fig. 6 A/B/C zeigt, wie die für die Therapie nicht benötigten Ribbons (radioaktive Strahler) innerhalb der Vorrichtung arretiert werden, wenn man die Kanäle am Kupplungs-Schieber mit Verschlußstiften nicht blind machen möchte.

Wie die Fig. 6 A zeigt, sind zwei Führungselemente 40 und 41 an dem magnetischen Betätigungselement (Nadel 32) jeweils auf beiden Seiten fest angebracht. Das biegsame Metalloder Plastikröhrchen, welches das radioaktive Material enthält, ist mit dem Führungselement 40 fest verbunden. Diese Führungselemente 40 und 41 sind im Gegensatz zum Betätigungselement 32 aus nicht magnetisierbarem Material. Sie werden benötigt, um das magnetische Betätigungselement 32 in ihrer Länge kurz zu halten. Das Führungselement 41 dient zum arretieren und zum Abgreifen der Endlage über den Mikroschalter 43.

Das Führungselement 40 ist zum Fixieren im Kupplungs-Schieber eingebaut, sowie zur optischen Kontrolle der Endstellung (Patient beladen oder Strahler herausgefahren) mit Hilfe der transparenten Schläuche 37, eingesetzt, da das Führungselement aus dem Rohr und dem Kupplungs-Schieber herausragt. Das Rohr 9 ragt durch die Platte 11 und ist mit dem Arretierblock 42, welcher zwischen Platte 11 und Deckel 4 angebracht ist, verbunden. Es können mehrere Rohre 9 in einer Arretiereinheit zusammengefaßt werden. In diesem Fällen sind die Rohre 9 von außen nicht verschlossen, sondern offen. Ein flexibler Drahtauslöser 44 (aus der Kamera-Technik bekannt), welcher den Arretierstift 45 enthält, ist mit dem Arretierblock 42 verbunden. Der Drahtauslöser 44 ist aus der Vorrichtung herausgeführt und vorzugsweise mit dem Schaltpult 48 verbunden, so daß mit der Taste 46 der Arretierstift 45 das Führungselement 41 formschlüssig oder kraftschlüssig (nicht dargestellt) arretieren kann.

Fig. 6 B zeigt, daß der Ribbon (Führungselement 41) nicht arretiert ist und somit mit dem Magneten 12 durch den Kupplungs-Schieber und durch den flexiblen und transparenten Schlauch aus der vorrichtung herausgefahren werden kann.

Fig. 6 C zeigt, daß der Ribbon formschlüssig arretiert ist und somit mit dem Magneten 12 nicht transportiert werden kann.

Wie in der Fig. 7 dargestellt, kann die ferngesteuerte Afterloading Vorrichtung (Rohr 1, Deckel 3) in ein transportables Gehäuse 47 integriert werden, wobei das Schaltpult 48 am transportablen Gehäuse angeschlossen werden kann. Um einen noch besseren Strahlschutz des Bedienungspersonals zu ermöglichen, kann die Vorrichtung mit einer Infrarot-Fernbedienung gesteuert werden, wobei der Empfänger 49 für die Infrarot-Fernbedienung an dem transportablen Gehäuse 47 angebracht wird.

## Patentansprüche

1. Afterloading Vorrichtung, insbesondere zur Therapie von Tumoren zum Transportieren von Ribbons mit radioaktivem Material mit einem Abschirmbehälter, bei der der Ribbon in einem Rohr (9) aus nichtmagnetisierbarem Material beweglich angeordnet ist, das sich in dem Abschirmbehälter befindet, dadurch gekennzeichnet, daß ein Magnet (12) entlang des Rohres bewegbar angeordnet ist, und ein magnetisierbares oder magnetisches Betätigungselement (32) mit dem Ribbon über ein Führungselement (40) so verbunden ist, daß der Ribbon durch den Magnet transportierbar ist und daß dem offenen Ende des Rohres (9) ein an- und abkoppelbarer und vom Patienten tragbarer Kupplungs-Schieber zugeordnet ist, welcher durch das Führungselement (40) das Betätigungselement (32) mit größerer Kraft als die Kraft, die zwischen Magnet und Betätigungselement vorhanden ist, festhalten kann.

2. Afterloading Vorrichtung nach Anspruch 1, dadurch gekennzeichnet, daß der Kupplungs-Schieber eine bewegliche Platte (28) umfaßt, die unter der Wirkung von einer oder mehreren Federn (29) steht und Bohrungen für den Durchtritt des Ribbons und des Führungselementes (40) enthält.

3. Afterloading Vorrichtung nach Anspruch 2, dadurch gekennzeichnet, daß dem Kupplungs-Schieber Verbindungsstifte (34) mit einer Bohrung zugeordnet sind, die die Verbindung zwischen Abschirmbehälter und Patient herstellen.

4. Afterloading Vorrichtung nach einem der Ansprüche 1 bis 3 dadurch gekennzeichnet, daß der Abschirmbehälter mehrere Rohre enthält und daß an jedem Rohr (9) ein Ringmagnet (12) sitzt.

5. Afterloading Vorrichtung nach Anspruch 4, dadurch gekennzeichnet, daß der Kupplungs-Schieber eine der Rohrzahl entsprechende Anzahl von Bohrungen aufweist.

6. Afterloading Vorrichtung nach Anspruch 1, dadurch gekennzeichnet, daß die Magnete (12) mit einer oder mehreren Halteplatten (14, 15) zusammengefaßt sind, die über einen Zahnriemen (16) und Zahnräder (17, 22) mit einem Antrieb verbunden sind.

7. Afterloading Vorrichtung nach Anspruch 1, dadurch gekennzeichnet, daß an einer bzw. beiden Seiten des magnetisierbaren Betätigungselementes (32) ein nicht magnetisierbares Führungselement (40) bzw. (41) angebracht ist.

8. Afterloading Vorrichtung nach Anspruch 7, dadurch gekennzeichnet, daß die Arretierung des Führungselementes (41) form- oder kraftschlüssig vorzugsweise in der Endlage erfolgt, wobei die Arretierungskraft größer sein muß als die Magnetkraft, die auf das Betätigungselement (32) wirkt.

9. Afterloading Vorrichtung nach Anspruch 1, dadurch gekennzeichnet, daß die Vorrichtung in einem transportablen Gehäuse (47) integriert ist.

10. Afterloading Vorrichtung nach Anspruch 9, dadurch gekennzeichnet, daß die Vorrichtung durch eine Infrarot-Fernbedienung gesteuert ist.

## Revendications

1. Dispositif d'afterloading (irradiation complémentaire), notamment pour le traitement de tumeurs, pour le transport de rubans portant un matériau radioactif et comportant un récipient de protection, dans lequel le ruban est disposé, de manière à être déplaçable, dans un tube (9) réalisé en un matériau non aimantable, qui est situé dans le récipient de protection, caractérisé par le fait qu'un aimant (12) est disposé de manière à être déplaçable dans le tube et qu'un élément d'actionnement aimantable ou magnétique (32) est relié au ruban par l'intermédiaire d'un élément de guidage (40) de telle sorte que le ruban peut être déplacé par l'aimant, et qu'à l'extrémité ouverte du tube (9) est associé un curseur d'accouplement qui peut être accouplé et désaccouplé et qui peut être porté par le patient et qui, au moyen de l'élément de guidage (40), peut maintenir fermement l'élément d'actionnement (32) avec une force supérieure à la force présente entre l'aimant et l'élément d'actionnement.

2. Dispositif d'afterloading suivant la revendication 1, caractérisé par le fait que le curseur d'accouplement comporte une plaque mobile (28), qui est soumise à l'action d'un ou de plusieurs ressorts (29) et comporte des perçages permettant le passage du ruban et de l'élément de guidage (40).

3. Dispositif d'afterloading suivant la revendication 2, caractérisé par le fait qu'au curseur d'accouplement sont associées des tiges de liaison (34) comportant un perçage, qui établissent la liaison entre le récipient de protection et le patient.

4. Dispositif d'afterloading suivant l'une des revendications 1 à 3, caractérisé par le fait que le récipient de blindage comporte plusieurs tubes et qu'un aimant annulaire (12) prend appui sur chaque tube (9).

5. Dispositif d'afterloading suivant la revendication 4, caractérisé par le fait que le curseur d'accouplement possède un nombre de perçages correspondant au nombre de tubes.

6. Dispositif d'afterloading suivant la revendication 1, caractérisé par le fait que les aimants (12) sont rassemblés avec une ou plusieurs plaques de retenue (14,15) et sont reliés à un dispositif d'entraînement.

7. Dispositif d'afterloading suivant la revendication 1, caractérisé par le fait qu'un élément de guidage non aimantable (40) ou (41) est disposé d'un côté ou sur les deux côtés de l'élément d'actionnement aimantable (32).

8. Dispositif d'afterloading suivant la revendication 7, caractérisé par le fait que le blocage de l'élément de guidage (41) est réalisé selon une liaison par formes complémentaires ou selon une liaison de force de préférence dans la position d'extrémité, la force de blocage devant être supérieure à la force magnétique qui agit sur l'élément d'actionnement (32).

9. Dispositif d'afterloading suivant la revendication 1, caractérisé par le fait que le dispositif est intégré dans un boîtier transportable (47).

10. Dispositif d'afterloading suivant la revendication 9, caractérisé par le fait que le dispositif est commandé par une telécommande à rayons infrarouges.

## Claims

1. An afterloading device for the treatment of tumours is shielded and is used to transport ribbons containing radioactive material, where ribbons are drawn along non-magnetic tubes (9) characterised in that a moveable magnet (12) is arranged around each tube. Connected to each ribbon is a metal pin (32) containing magnetic material. The magnet transports the ribbon connected to a leader (49) by magnetic attraction along the tube (channel) out through the open end (9). At this point a portable coupler grips the pins and thus the leader (40) with a force greater than the attraction between the magnet and pin (32). This allows the coupler to be detached (or reattached), separating (or re-joining) the patient and machine.

2. The afterloading device under 1 is characterised by the moveable perforated plate (28) in each coupler which is actuated by spring action (29). The perforations allow access for the ribbons and leaders (40).

3. The afterloading device under 2 is characterised by conduits (34), one for each pin, which create the connection between the shielded machine and patient.

4. The afterloading device under 1 to 3 is characterised by the fact that the shielded machine contains multiple channels and each channel (9) has an annular magnet (12).

5. The afterloading device as under 4 is characterised by the coupler which has the same number of channel perforations as the conduits.

6. The afterloading device as under 1 is characterised by the magnets (12) held by one or more plates (14, 15) and connected to a drive system.

7. The afterloading device as under 1 is characterised by non-magnetic leaders (40, 41) on one side of the magnetic pin (32).

8. The afterloading device as under 7 is characterised by the forced pin stop (41) whereby the stopping force must be greater than the magnetic force on the pin (32).

9. The afterloading device as under 1 is characterised by the transport trolley (47).

10. The afterloading device as under 9 is characterised by the remote infrared control.

Fig. 1:
Schnittansicht einer ferngesteuerten
Afterloading Vorrichtung

Fig. 2: vergrößerte Darstellung des
Ribbons in Rohr 9

Fig. 3: vordere Ansicht der
Afterloading Vorrichtung mit
geöffnetem Deckel

Fig.4: Schnittansicht eines Kupplungs-Schiebers für Afterloading Vorrichtung

Fig.5: vordere Ansicht des Kupplungs - Schiebers

EP 0 185 939 B1

2

Fig. 6 A: Schematische Darstellung der
Arretierung von Strahler (Ribbon)

Fig. 6 B: Strahler nicht
arretiert

Fig. 6 C: Strahler
arretiert

Plastikröhrchen

EP 0 185 939 B1

Fig. 7: Schematische Darstellung der ferngesteuerten
Afterloading Vorrichtung integriert in einem
Wagen.